Europäisches Patentamt

(19) European Patent Office

Office européen des brevets

(11) Numéro de publication : **0 294 298 B1**

(12) # FASCICULE DE BREVET EUROPEEN

(45) Date de publication du fascicule du brevet :
**29.05.91 Bulletin 91/22**

(51) Int. Cl.⁵ : **A61F 2/38**

(21) Numéro de dépôt : **88420164.1**

(22) Date de dépôt : **20.05.88**

(54) **Prothèse totale de genou.**

(30) Priorité : **21.05.87 FR 8707353**

(43) Date de publication de la demande :
**07.12.88 Bulletin 88/49**

(45) Mention de la délivrance du brevet :
**29.05.91 Bulletin 91/22**

(84) Etats contractants désignés :
**DE ES FR GB IT**

(56) Documents cités :
**FR-A- 2 568 467**
**GB-A- 2 067 412**
**US-A- 3 840 905**
**JOURNAL OF BONE AND JOINT SURGERY,**
**vol. 64-A, no. 9, décembre 1982, pages**
**1317-1323, Boston, Massachusetts, US; J.N.**
**INSALL et al.: "The posterior stabilized**
**condylar prosthesis: a modification of the total**
**condylar design"**

(73) Titulaire : **Etablissements TORNIER**
**Chemin Doyen Gosse B.P. 11**
**F-38330 Saint Ismier (FR)**
Titulaire : **Dejour, Henri**
**21, avenue des deux fermes**
**F-69500 Bron (FR)**
Titulaire : **Chambat, Pierre**
**Villette de Vienne**
**F-38200 Vienne (FR)**
Titulaire : **Deschamps, Gérard**
**Clos Morelle**
**F-71640 Givry (FR)**

(72) Inventeur : **Tornier, Alain**
**Chemin Doyen Gosse**
**F-38330 Saint Ismier (FR)**

(74) Mandataire : **Karmin, Roger et al**
**Cabinet MONNIER 150, Cours Lafayette**
**F-69003 Lyon (FR)**

EP 0 294 298 B1

**Description**

La présente invention est relative à des perfectionnements apportés aux prothèses totales du genou comportant à la manière connue une pièce fémorale et un plateau tibial. La pièce fémorale est utilisée pour remplacer une partie des condyles détruits, tandis que le plateau tibial se substitue à la partie supérieure endommagée du tibia.

Il existe déjà des prothèses du genre en question qui sont tout d'abord les prothèses à glissement dans lesquelles la pièce fémorale glisse sur une zone de faible surface par rapport à l'élément tibial. Il existe aussi des prothèses à charnières ou à mouvements contrôlés, le mouvement des éléments est pré-déterminé, ce qui peut induire de forte contraintes sur eux. Toutefois, de telles prothèses ne donnent qu'une mobilité moyenne au genou dans le cas de grande destruction.

On connaît par le document GB-A-2 067 412 une prothèse de genou selon les caractéristiques du préambule de la revendication 1. L'élément fémoral du document GB-A-2 067 412 est pourvu entre ses deux condyles d'un passage se terminant par une came convexe. L'élément tibial ou plateau de la prothèse considérée, qui comporte des empreintes de réception des condyles, est muni d'une rampe concave s'élevant vers l'élément fémoral et pénétrant largement entre ses deux condyles.

Lors de la coopération de la came et de la rampe après un déplacement angulaire libre, il se crée des contraintes importantes du fait du bras de levier important crée par rapport à la fixation du plateau tibial.

De plus, l'importance de la pénétration de la rampe du plateau tibial limite considérablement l'amplitude de la rotation du tibia par rapport au fémur.

Enfin la hauteur de la rampe du plateau tibial entraîne une resection importante de l'épiphyse du fémur et qui bien entendu diminue considérablement sa résistance.

Les perfectionnements qui font l'objet de la présente invention visent à remédier aux inconvénients ci-dessus et à permettre de réaliser une prothèse totale du genou n'induisant que des contraintes minimales entre ses éléments, tout en assurant une excellente mobilité du genou. L'invention vise encore à réduire au maximum la resection de l'épiphyse fémorale.

La prothèse suivant l'invention est caractérisée en ce que les deux condyles sont asymétriques, en ce que la came convexe est réalisée sous la forme d'un troisième condyle qui fait suite à une piste horizontale située en dessous du niveau des empreintes et en ce que le point de contact du troisième condyle contre la rampe concave affectant la forme d'une butée courbe se trouve à une faible distance du plateau tibial sur toute la course de contact de ces deux éléments de manière à diminuer au maximum les contraintes sur ledit plateau tibial.

Du fait de la position basse du troisième condyle, on diminue au maximum le bras de levier créé par son action contre la butée du plateau tibial, de sorte que les contraintes transmises au tibia sont minimes.

Le dessin annexé, donné à titre d'exemple, permettra de mieux comprendre l'invention, les caractéristiques qu'elle présente et les avantages qu'elle est susceptible de procurer :

Fig. 1 est une vue éclatée en perspective des deux éléments d'une prothèse de genou établie conformément à l'invention, le fémur et le tibia ayant été représentés schématiquement en traits discontinus.

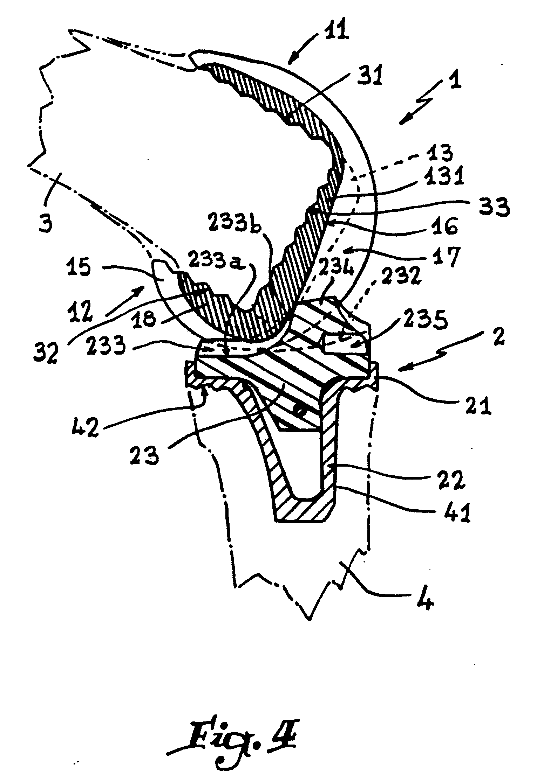

Fig. 2 est une vue de la pièce fémorale suivant la flèche F de fig. 1.

Fig. 3 à 5 montrent diverses positions des deux éléments de la prothèse suivant l'invention, respectivement en position debout, en flexion légère et en flexion maximale du genou.

Fig. 6 est une coupe suivant VI-VI (fig. 5).

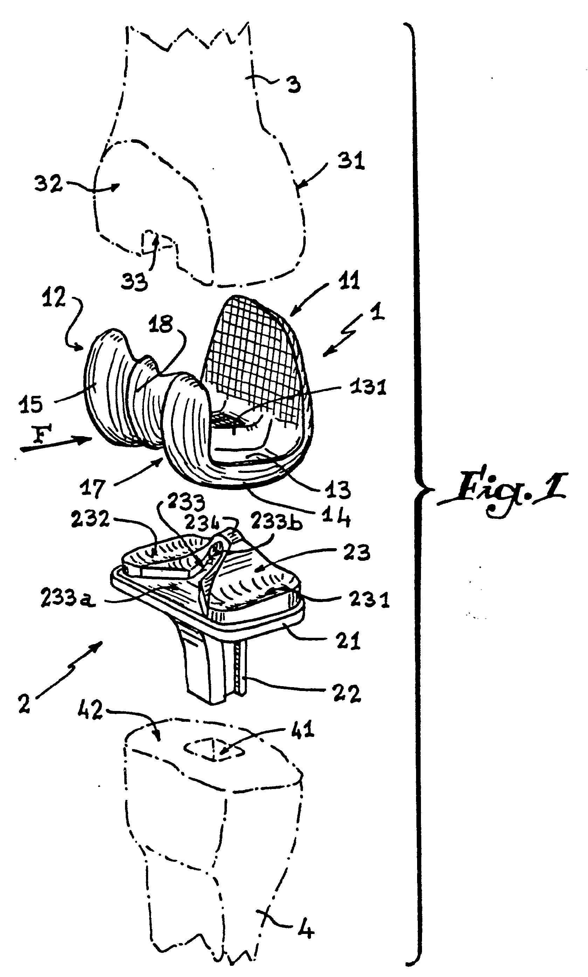

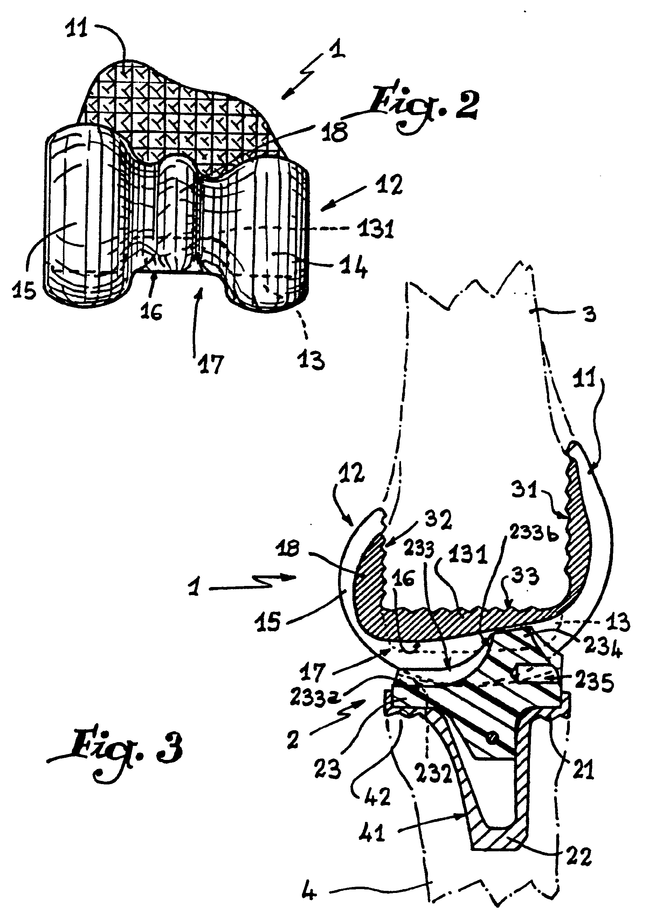

La prothèse totale du genou suivant l'invention comprend essentiellement, comme illustré en fig. 1, une pièce fémorale 1 et un plateau tibial 2.

La pièce fémorale 1 comporte en section transversale la forme générale d'un U, c'est-à-dire qu'elle est formée d'une première aile antérieure 11 et d'une seconde aile postérieure 12. Les faces internes grossièrement planes de ces deux ailes sont destinées à coopérer avec des méplats 31, 32 ménagés au moyen de découpes antérieure et respectivement postérieure réalisées sur l'épiphyse du fémur 3. Le voile de liaison 13 des deux ailes 11 et 12 de la pièce fémorale 1 est généralement plat, mais il comporte toutefois en son centre une saillie transversale 131 pour des raisons qu'on expliquera mieux plus loin. Lors de la mise en place de la pièce fémorale 1 par rapport à l'épiphyse du fémur 3, ladite saillie s'engage dans une saignée 33 de ladite épiphyse correspondant à une faible resection de celle-ci.

La face externe des deux ailes et du voile transversal de la pièce fémorale 1 comporte deux condyles latéraux asymétriques 14 et 15 à la manière des prothèses usuelles telles que celles décrites dans le document GB-A-2 067 412.

Le plateau tibial 2 est réalisé sous la forme d'une plaque métallique 21 substantiellement plane pourvue d'une queue 22 destinée à être engagée dans une perforation 41 pratiquée dans l'extrémité supérieure tronquée 42 du tibia 4 dans laquelle il est scellé, la face inférieure de la plaque 21 venant appuyer sur ladite face 42. Le dessus de la plaque 21 reçoit une couche de matière plastique à très faible coefficient de frottement de qualité bien connue dans la pratique et référencée de manière générale 23. La face apparente de la couche 23 est creusée de deux empreintes 231, 232 avec lesquelles coopèrent à la manière usuelle les condyles 14 et 15 de la pièce fémorale 1, pour permettre une rotation convenable du genou artificiel.

Conformément à l'invention, la pièce fémorale 1 est pourvue sur sa face extérieure, au niveau de son voile et entre ses deux condyles latéraux 14 et 15, d'une facette 16 à fond plat. Ainsi, les deux condyles et celle-ci déterminent une gorge 17 à section générale concave qui correspond à la saillie 131 du voile 13 de la pièce en question. L'aile 12 de cette dernière est pourvue sur sa face extérieure d'un troisième condyle central 18 réalisé sous la forme d'une portion de tore.

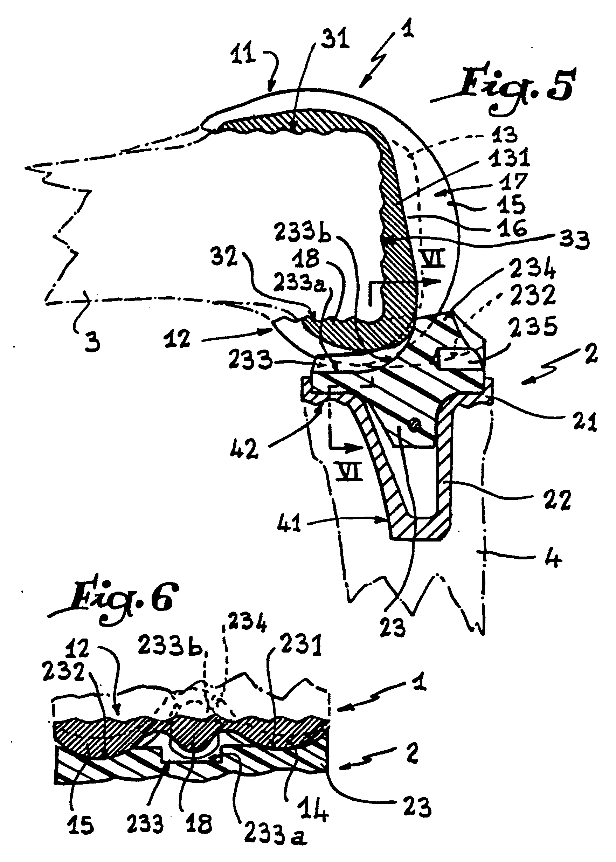

La couche 23 du plateau tibial 2 est munie dans sa partie médiane située entre les deux empreintes 231 et 232 d'une rampe 233 qui comporte tout d'abord une piste horizontale 233a (fig. 3) se prolongeant vers l'extrémité antérieure dudit plateau par une butée courbe 233b qui se dresse à l'opposé du tibia 4. Cette piste se trouve à un niveau inférieur à celui des empreintes 231, 232. La butée 233b est constituée par la paroi courbe d'une protubérance 234 dont la partie antérieure est pourvue d'un trou 235 destiné à l'éventuelle mise en place d'une rotule non représentée. Le fonctionnement est le suivant :

En position debout comme illustré en fig. 3, les condyles 15 et 16 de la pièce fémorale 1 sont en contact avec les empreintes 231 et 232 du plateau tibial 2. On observe que le dessus de la protubérance 234 n'est pas en contact avec la facette 16 de la pièce fémorale 1.

Lors d'une flexion de faible amplitude, la rotation relative de la pièce fémorale et du plateau tibial s'effectue de manière que la protubérance 234 se déplace librement dans la gorge 17. A partir d'une flexion de plus grande importance, soit à partir d'environ 60°, le troisième condyle 18 vient en contact contre la butée 233b (fig. 4). L'action de celle-ci contre la came que constitue le troisième condyle 18 entraîne un report en arrière du centre de rotation du genou pour obtenir une bonne flexion, tandis que ce contact évite toute luxation antérieure du genou (fig. 4). Si le mouvement de flexion se prolonge au-delà de 60° (fig. 5), le troisième condyle continue à coopérer avec la butée courbe 133b pour produire les mêmes effets que ceux indiqués plus haut.

La faible hauteur de la protubérance 234 permet un degré de rotation satisfaisant au cours de la flexion assurant un fonctionnement plus logique et un jeu ligamentaire périphérique plus proche de celui d'un genou normal.

Du fait que le point de contact du troisième condyle 18 contre la butée courbe 233b se trouve à une faible distance du plateau tibial sur toute la course de contact de ces deux éléments, on diminue au maximum les contraintes transmises au tibia. Autrement dit, le bras de levier agissant sur le système est très court.

On a ainsi réalisé une prothèse totale du genou qui permet d'améliorer l'amplitude de flexion du genou sans risque de dislocation, d'obtenir un meilleur résultat fonctionnel par l'amélioration de l'efficacité du système quadricipital et enfin de diminuer les contraintes sur l'articulation fémoro-patellaire qui n'a plus en charge de lutter contre le tiroir postérieur du tibia au cours de la flexion.

Enfin la faible hauteur de la protubérance permet de n'effectuer qu'une légère resection de l'épiphyse fémorale.

**Revendications**

1. Prothèse totale de genou du genre comprenant une pièce fémorale (1) pourvue de deux condyles (14, 15) un externe, un interne, et un plateau tibial (2) muni d'empreintes (231, 232) propres à recevoir lesdits condyles qui sont séparés par une gorge (17) à laquelle fait suite une came convexe disposée entre les parties postérieures desdits condyles, le plateau tibial (2) comportant entre ses empreintes une rampe concave (233) avec laquelle la came vient coopérer après une flexion libre de la prothèse, caractérisée en ce que les deux condyles sont asymétriques, en ce que la came convexe est réalisée sous la forme d'un troisième condyle (18) qui fait suite à une piste horizontale (233a) située en dessous du niveau des empreintes (231, 232) et en ce que le point de contact du troisième condyle (18) contre la rampe concave affectant la forme d'une butée courbe (233b) se trouve à une faible distance du plateau tibial sur toute la course de contact de ces deux éléments de manière à diminuer au maximum les contraintes sur ledit plateau tibial.

**Ansprüche**

1. Totale Kniegelenkprothese mit einem Schenkelteil (1), das mit zwei Gelenkköpfen (14, 15), nämlich einem äußeren und einem inneren, ausgestattet ist, und mit einer tibialen Platte (2), die mit Vertiefungen (231, 232) versehen ist, die die Gelenkhöpfe aufnehmen können, welche getrennt sind voneinander durch eine Hohlkehle (17), auf die ein konvexer Kamm folgt, der zwischen den hinteren Abschnitten dieser Gelenkköpfe angeordnet ist, wobei die tibiale Platte (2) zwischen ihren Vertiefungen eine konkave Rampe (233) aufweist, mit der der Kamm nach einer freien Beugung der Prothese kooperiert, dadurch **gekennzeichnet,** daß die beiden Gelenkköpfe asymmetrisch sind, daß der konvexe Kamm in Form eines dritten Gelenkkopfes (18) ausgestaltet ist, der einer horizontalen Bahn (233a) folgt, die unterhalb der Höhe der Vertiefungen (231, 232) angeordnet ist, und daß sich der Kontaktpunkt des dritten Gelenkkopfes (18) an der konkaven Rampe, welche die Form eines gekrümmten Anschlags (233b) annimmt, in einem geringen Abstand von der tibialen Platte befindet, und zwar solange diese beiden Elemente miteinander in Kontakt sind, so daß die auf die tibiale Platte einwirkenden Belastungen möglichst gering sind.

## Claims

1. A complete knee prosthesis comprising a femoral part (1) provided with two condyles (14, 15), one external and one internal, and a tibial plate (2) provided with recesses (231, 232) suitable for receiving the said condyles which are separated by a channel (17) which is followed by a convex cam disposed between the posterior parts of the said condyles, the tibial plate (2) comprising a concave ramp (233) between its recesses, with the cam acting together with the ramp after a free flexure of the prosthesis, characterised in that the two condyles are asymmetric, and that the convex cam takes the form of a third condyle (18) which follows a horizontal track (233a) situated below the level of the recesses (231, 232), and in that the point of contact of the third condyle (18) with the concave ramp takes the form of a curved spacer (233b) and is located at a short distance from the tibial plate over the whole path of contact of these two components in such a way as to provide the maximum reduction of stress on the said tibial plate.

*Fig.1*

*Fig. 2*

*Fig. 3*

*Fig. 4*

*Fig. 5*

*Fig. 6*